# EUROPEAN PATENT APPLICATION

(11) **EP 4 600 908 A1**
(43) Date of publication of application: **13.08.2025**
(21) Application number: 25156311.0
(22) Date of filing: 06.02.2025
(51) Int. Cl.: G06T 11/00, A61B 6/02, A61B 6/03, A61B 6/40, G01N 23/046

(54) **METHOD AND SYSTEM FOR CORRECTING CONE BEAM SCAN CT FOR INTENSITY DROP-OFF**

(30) Priority: 09.02.2024 US 202463551730 P
(71) Applicant: Carl Zeiss X-Ray Microscopy, Inc., Dublin, California 94568 (US)
(72) Inventor: Yang, Faguo, Dublin, 94568 (US); Andreyev, Andriy, Dublin, 94568 (US); Andrew, Matthew, Dublin, 94568 (US)
(74) Representative: Gulde & Partner

(57) **Abstract**

A method for cone beam CT intensity correction such as for circular and other scanning geometries. The method comprises determining an intensity compensation for a selected scanning configuration and applying the intensity compensation to a reconstructed volume of interest to produce an intensity compensated volume, which is used to scale the volume of interest.

## Description

### BACKGROUND OF THE INVENTION

X-ray micro tomography systems provide high-resolution, non-destructive imaging of internal structures in samples. The systems are utilized in a variety of industrial and research applications, such as mining, manufacturing, materials science, clinical research, and failure analysis, in examples. The systems provide the ability to visualize features in samples without the need to cut and slice the samples.

X-ray micro tomography systems include X-ray projection systems that produce projection data of the sample and computer systems that reconstruct tomographic volumes of the sample from the projection data. In operation, the X-ray projection system executes a scan of a sample at different angles to produce the projection data. During a scan, X-rays are directed to the sample, and are absorbed or scattered by the sample as the X-rays travel through the sample. The X-rays not absorbed or scattered away are transmitted through and modulated by the sample. A detector system receives the transmitted X-rays, and creates an image representation, in pixels, of the received X-rays. The series of these X-ray projections at different angles produced from the scan form the projection data of the sample. Then, the computer system accesses the projection data, and applies tomographic reconstruction algorithms to the projection data to reconstruct volume datasets of the sample. A volume dataset is a three-dimensional (3D) representation of the entire sample, and a slice is a two dimensional (2D) cross-sectional image of the sample based on the volume dataset.

Often, a circular scanning configuration/acquisition geometry is used. In this arrangement, the sample is rotated, typically around a vertical axis and a series of projections are obtained at different angles. Then the sample's height is changed and a new set of projections are obtained. It has the advantage of mechanical simplicity. There is the problem of data insufficiency, however. This scanning configuration will not acquire enough raw data to exactly recover attenuation coefficients of a 3D sample object. An obvious image artifact is an intensity drop off along the longitude direction in a high cone-beam region. It thus exhibits image artifacts related to the cone beam and axial variation of the object anatomy. Therefore, non-circular scanning geometries such as circle plus a line, two circles, helix or circular sinusoid are often employed when more accurate images free of cone beam artifacts are desired, despite the added mechanical complexity.

The most commonly used reconstruction algorithms fall into a class of reconstruction techniques termed analytical reconstruction. The objective is to find a closed-form solution to the problem of reconstructing an object's internal structure from its projections. The most common analytical method is filtered back projection (FBP). The projections are first processed using a high-pass filter, usually a ramp filter, in the frequency or space domain. Then, each filtered projection is "smeared" back onto the imaging plane as if each data point emits back uniformly in the shape of the original beam. These back projections from all angles are summed up to produce the reconstructed volume, which approximates the object's internal structure. The filtering and back projection operations collectively help in obtaining a more accurate and less blurred reconstruction of the original object. One type of filtered back projection is FDK. It is used often with X-ray micro tomography systems since it reduces artifacts associated with the typical cone-shaped beam. See Feldkamp, L.A., Davis, L.C. and Kress, J.W. (1984) Practical Cone-Beam Algorithm. Journal of the Optical Society of America A, 1, 612-619.

### SUMMARY OF THE INVENTION

The present invention concerns a method and system to correct intensity-drop-off image artifacts caused by different trajectories in cone-beam CT scanners.

Cone-beam circular trajectory is very popular in medical and industrial CT scans due to its convenience and the simplicity of this acquisition geometry.

The present invention can also be applied to other scan trajectories such as circle plus a line, sine wave, variable magnification (elliptical trajectory), two circles. Here, the present invention can be used to deal with insufficient raw data leading to image artifacts.

In a preferred embodiment, a coefficient map is created for intensity compensation. This map is inverted and then multiplied by the reconstructed volume of interest. This increases the portions of volume that were under-sampled.

In general, according to one aspect, the invention features a method for cone beam CT intensity correction. The method comprises determining an intensity compensation for a selected scanning configuration by forward projecting using the selected scanning configuration, performing a reconstruction of forward projected data and using reconstructed data to generate the intensity compensation and applying the intensity compensation to a reconstructed volume of interest to produce an intensity compensated volume.

In the present embodiment, the preferred scanning configuration is a circular scanning configuration although other suitable scanning configurations can be selected (circle and line, ellipse, sine wave, double circle, etc.). The forward projection, such as volume that is instantiated with each voxel being filled with a constant (i.e., a constant value, such as e.g., a constant intensity, brightness or density value), should have the same dimensions and voxel size as the reconstructed volume of interest. A reconstruction of forward projected data is then performed and using reconstructed data to generate the intensity compensation. The volume filled with a constant as well as the forward projection of said volume may be simulated or obtained by the scanning configuration without using a sample or with using a reference sample having a constant density over the whole sample volume.

In the illustrated example, a voxel-by-voxel intensity compensation is generated by inverting the reconstructed forward projected data. The reconstructed volume of interest and the reconstruction of forward projected data are performed using FDK.

In general, according to another aspect, the invention features an x-ray tomography system, comprising an x-ray system including an x-ray source system for generating an x-ray beam and a detector system for detecting the x-ray beam after transmission through a sample to generate projection data. A computer system determines an intensity compensation for a selected scanning configuration by forward projecting using the selected scanning configuration, performing a reconstruction of forward projected data and using reconstructed data to generate the intensity compensation, and applying the intensity compensation to a reconstructed volume of interest to produce an intensity compensated volume.

The above and other features of the invention including various novel details of construction and combinations of parts, and other advantages, will now be more particularly described with reference to the accompanying drawings and pointed out in the claims. It will be understood that the particular method and device embodying the invention are shown by way of illustration and not as a limitation of the invention. The principles and features of this invention may be employed in various and numerous embodiments without departing from the scope of the invention.

### BRIEF DESCRIPTION OF THE DRAWINGS

In the accompanying drawings, reference characters refer to the same parts throughout the different views. The drawings are not necessarily to scale; emphasis has instead been placed upon illustrating the principles of the invention. Of the drawings:
Fig. 1 is a schematic diagram of an X-ray micro tomography system to which the methods of the present invention are applicable;
Fig. 2A and 2B are a slice along the longitudinal direction of a water phantom and a plot of gray values as a function of distance along line A-A of showing the intensity drop-off;
Fig. 3 is a flow diagram showing a method based on FDK (filtered back projection reconstruction) to correct the intensity-drop-off image artifacts caused by circular trajectories of cone-beam CT scanners;
Fig. 4A and 4B are a slice along the longitudinal direction of a water phantom and a plot of gray values as a function of distance along line A-A of showing a reduced intensity drop-off associated with the present invention.

### DETAILED DESCRIPTION OF THE PREFERRED EMBODIMENTS

The invention now will be described more fully hereinafter with reference to the accompanying drawings, in which illustrative embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

As used herein, the term "and/or" includes any and all combinations of one or more of the associated listed items. Further, the singular forms and the articles "a", "an" and "the" are intended to include the plural forms as well, unless expressly stated otherwise. It will be further understood that the terms: includes, comprises, including and/or comprising, when used in this specification, specify the presence of stated features, integers, steps, operations, elements, and/or components, but do not preclude the presence or addition of one or more other features, integers, steps, operations, elements, components, and/or groups thereof. Further, it will be understood that when an element, including component or subsystem, is referred to and/or shown as being connected or coupled to another element, it can be directly connected or coupled to the other element or intervening elements may be present.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

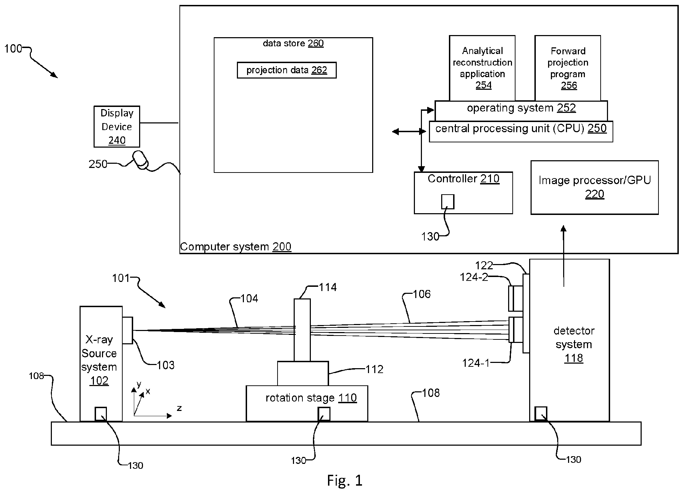

Fig. 1 is a schematic diagram of an X-ray micro tomography system 100 to which the methods and workflows of the present invention are applicable.

In general, the X-ray micro tomography system 100 combines an X-ray microscopy system 101 and a computer system 200 for receiving projections and calculating volume datasets from those projections.

The X-ray microscopy system includes the X-ray source system 102 that generates an X-ray beam 104 and a rotation stage 110 with sample holder 112 for holding and rotating and translating the sample 114 in the X-ray beam 104 from the X-ray source system 102. Images or X-ray projections are captured by the detector system 118. The X-ray source system 102, the rotation stage 110, and the detector system 118 are mounted to a base 108 of the X-ray CT system 100. A computer system 200 typically receives and processes these projections and provides general control of the system 100. The computer system 200 or other computer will typically perform tomographic reconstruction using the X-ray projections to create volume datasets.

The X-ray source 102, in one example, is a laboratory X-ray source because of its ubiquity and relatively low cost. Such sources typically produce a cone-beam, polychromatic X-ray beam.

Common laboratory X-ray sources include an X-ray tube, in which electrons are accelerated in a vacuum by an electric field and shot into a target piece of metal, with X-rays being emitted as the electrons decelerate in the metal.

In one example, the X-ray source 102 is a micro-focused source, with a Tungsten target. Targets that include Molybdenum, Gold, Platinum, Silver or Copper also can be employed. Preferably, a transmissive-type target configuration of the X-ray source 102 is used in which the electron beam strikes the thin target 103 from its backside. The X-rays emitted from the other side of the target 103 are then used as the beam 104.

When the sample 114 is exposed to the X-ray beam 104, the X-ray photons transmitted through the sample form an attenuated X-ray beam 106 that is received by the detector system 118.

In the most common configuration of the detector system 118, a magnified projection image of the sample 114 is formed on the detector system 118 with a geometrical magnification that is equal to the inverse ratio of the source-to-sample distance and the source-to-detector distance. Generally, the geometrical magnification provided by the X-ray stage is between 2x and 100x, or more. In this case, the resolution of the X-ray image is limited by the focus spot size or virtual size of the X-ray source system 102.

To achieve high resolution, an embodiment of the X-ray micro tomography system 100 further utilizes a very high resolution detector 124-1 of the detector system 118 in conjunction with positioning the sample 114 close to the X-ray source system 102. In one implementation of the high-resolution detector 124-1, a scintillator is used in conjunction with a microscope objective to provide additional magnification in a range between 2x and 100x, or more.

Other possible detectors can be included as part of the detector system 118 in the illustrated X-ray CT system 100. For example, the detector system 118 can include a lower resolution detector 124-2. This could be a flat panel detector or a detector with a lower magnification microscope objective, in examples. Configurations of one, two, or even more detectors 124 of the detector system 118 are possible.

Often, two or more detectors 124-1, 124-2 are mounted on a rotating turret 122 of the detector system 118, so that they can be alternately rotated into the path of the attenuated beam 106 from the sample 114.

Typically, based on operator defined parameters, the controller 210 of the computer system 200 instructs the rotation stage 110 via the control interface 130 to position the sample 114 and the detector 124 for the desired CT set up. After completion, the controller 210 rotates the sample 114 relative to the beam 104 to perform the CT scan of the sample 114 and saving the projection data 262 to a datastore 260. The scanning configuration commanded by the controller 210 to the rotation stage might be circular or another scanning configuration such as circle and line, ellipse, sine wave, double circle, etc.

In one example, the computer system 200 includes a graphics or other acceleration processor 220 that analyzes the X-ray projections and possibly performs the calculations necessary for tomographic reconstructions created from the X-ray projections. A display device 240, connected to the computer system 200, displays information from the X-ray CT system 100 and also typically the projection data, volume data and slices. An input device 250 such as a touch screen, keyboard, and/or computer mouse enables interaction between the operator, the computer system 200, and the display device 240.

Via user interface applications executing on the computer system 200 that display their interfaces on the display device 240, in one example, the operator defines/selects CT scan or calibration. These include X-ray acceleration voltage settings, and settings for defining the X-ray energy spectrum of the scan and exposure time on the X-ray source system 102. The operator also typically selects other settings such as the number of X-ray projection images to create for the sample 114, the angles to rotate the rotation stage 110 including the scanning configuration (i.e., circular, circle and line, ellipse, sine wave, double circle, or other).

The computer system 200, with the assistance of its image processor 220, accepts the image or projection information from the detector system 118 associated with each rotation angle of the sample 114. Often the image processor 220 combines the projection data using reconstruction algorithms to create 3D tomographic reconstructed volume information for the sample (volume datasets).

The computer system 200 will typically execute a reconstruction application 254 for reconstructing the projection data 262 such as one implementing the FDK algorithm. Also provided is a forward projection program 256. Both the reconstruction application 254 and the forward projection program 256 are stored in a non-transitory storage medium such as an electronic or magnetic storage medium.

The reconstruction applications 254 runs on top of the operating system 252. The operating system 252, in turn, is executed by the computer's central processing unit(s) (CPU) 250.

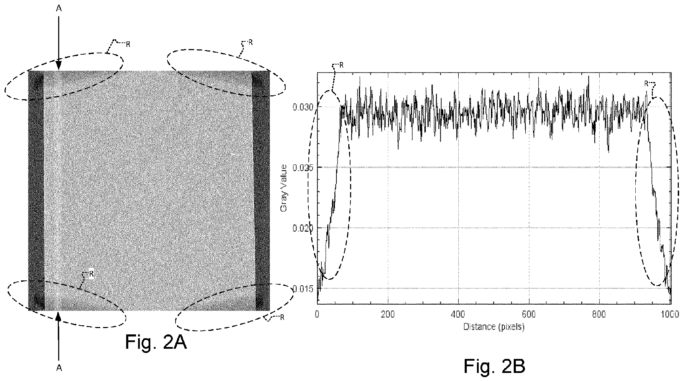

Figs. 2A and 2B illustrate a fundamental problem with the circular scanning configuration and similar configurations.

Fig. 2A is a slice along the longitudinal direction of a water phantom that was generated using a circular scanning configuration. In each of the corners, there is a marked drop-off in intensity. See highlighted regions R, for example.

Fig. 2B is a plot of gray values as a function of distance along line A-A. In the highlighted regions, the intensity drop-off R is notable.

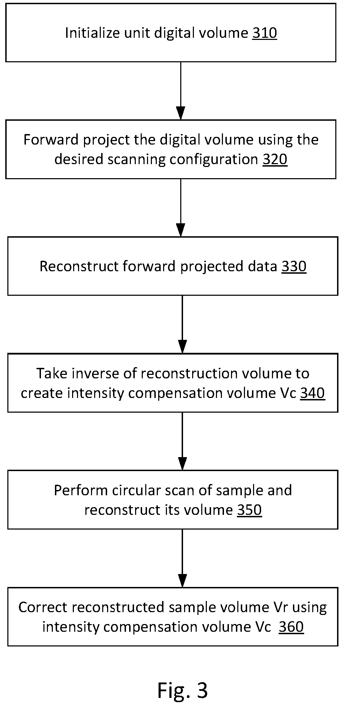

Fig. 3 is a flow diagram showing a method to correct the intensity-drop-off image artifacts caused by trajectories in cone-beam CT scanners such as X-ray micro tomography systems as described above and also in medical CT scanners in which the patient is supported by the source and detector system are rotated around the patient.

In step 310, a 3D image volume V1, which has the same dimensions and voxel size as the reconstructed 3D image volume, is instantiated. In this initialized volume, each voxel is filled with a constant denote by C corresponding to the desired reconstructed field of view (FOV) for the system geometry and based on a selected scanning configuration. In the illustrated embodiment, the selected scanning configuration is a circular scanning configuration, although other scanning configurations could be selected that exhibit intensity drop-off due to a cone-shaped x-ray beam, such as scanning configurations: circle and line, ellipse, sine wave, double circle, etc.

In step 320, the constant-number-filled image volume V1 is forward projected using the same geometry of each view as the original scan, which is executed on a computer using a forward projection computer program 254. These forward projected digital images are denoted by P1.

In step 330, the forward-projected data P1 is then put to the FDK method to perform a reconstruction using the analytical reconstruction application 254. The reconstructed volume is denoted by V2.

In step 340, an intensity compensation volume Vc is created by inversing volume V2, element (voxel) by element (voxel). Specifically, for each element, Vc=C/V2.

In step 350, the raw projection data acquired during the circular or other appropriate (circle and line, ellipse, sine wave, double circle) scanning configuration/acquisition geometry of the sample 114 is reconstructed using the FDK method by the analytical reconstruction application 254. This creates a reconstructed volume of interest of the sample 114. This reconstructed volume dataset of the sample 114 is denoted by Vr. It should be noted that especially in connection with this step, order is not important. In fact, this data acquisition of the sample is in practice often performed at the very beginning (prior to step 310) and then the correction is generated per that acquisition geometry as outlined in steps 310-340.

Finally, in step 360, the raw reconstructed volume Vr is multiplied by the intensity compensation volume Vc element/voxel-wise to obtain the final intensity compensated reconstructed volume Vrc, corrected for intensity drop-off.

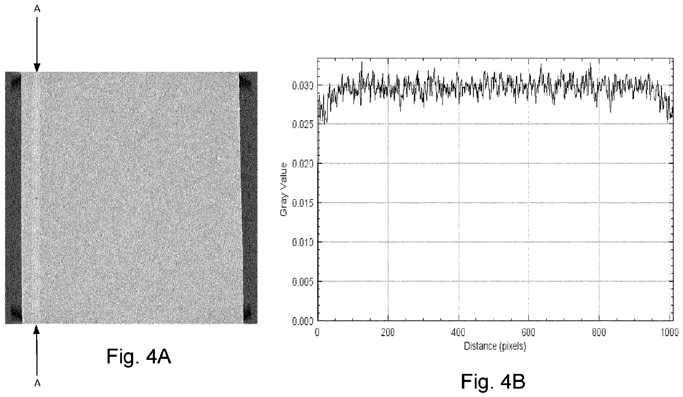

Figs. 4A and 4B illustrate the correction for intensity drop-off for the circular scanning configuration.

Fig. 4A is a slice along the longitudinal direction of a water phantom generated using a circular scanning configuration but corrected for intensity drop-off. The corners no longer exhibit the drop-off in intensity.

Fig. 4B is a plot of gray values as a function of distance along line A-A. The intensity drop-off is no longer present.

While this invention has been particularly shown and described with references to preferred embodiments thereof, it will be understood by those skilled in the art that various changes in form and details may be made therein without departing from the scope of the invention encompassed by the appended claims.

## Claims

1. A method for cone beam CT intensity correction, comprising:
determining an intensity compensation for a selected scanning configuration by forward projecting using the selected scanning configuration;
performing a reconstruction of forward projected data and using reconstructed data to generate the intensity compensation; and
applying the intensity compensation to a reconstructed volume of interest to produce an intensity compensated volume.

2. The method of claim 1, wherein the selected scanning configuration is a circular or other suitable scanning configuration, the other suitable scanning configuration being preferably one of circle and line, ellipse, sine wave, and double circle.

3. The method of either of claims 1 or 2, wherein the forward projection has the same dimensions and voxel size as the reconstructed volume of interest.

4. The method of any one of claims 1 to 3, wherein the forward projection is performed of a volume that is instantiated with each voxel being filled with a constant.

5. The method of claim 4, wherein a voxel-by-voxel intensity compensation is generated by inverting the reconstructed forward projected data.

6. The method of any of claims 4 or 5, wherein the reconstructed volume of interest and the reconstruction of forward projected data are performed using FDK.

7. A computer software product for cone beam CT intensity correction comprising instructions which, when the instructions are executed by a computer, cause the computer to carry out the method of any of claims 1 to 6.

8. The computer software product of claim 7, stored in a non-transitory storage medium such as an electronic or magnetic storage medium.

9. An x-ray tomography system (100), comprising:
an x-ray microscopy system (101) including an x-ray source system (102) for generating an x-ray beam (104) and a detector system (118) for detecting the x-ray beam (104) after transmission through a sample (114) to generate projection data; and
a computer system (200) for determining an intensity compensation for a selected scanning configuration by forward projecting using the selected scanning configuration, performing a reconstruction of forward projected data and using reconstructed data to generate the intensity compensation, and applying the intensity compensation to a reconstructed volume of interest to produce an intensity compensated volume.

10. The system (100) of claim 9, wherein the selected scanning configuration is a circular or other suitable scanning configuration, wherein the other suitable scanning configuration is one of circle and line, ellipse, sine wave, and double circle, etc.

11. The system (100) of either of claims 9 or 10, wherein the forward projection has the same dimensions and voxel size as the reconstructed volume of interest.

12. The system (100) of any one of claims 9 to 11, wherein the forward projection is performed of a volume that is instantiated with each voxel being filled with a constant.

13. The system (100) of claim 12, wherein a voxel-by-voxel intensity compensation is generated by the computer system (200) by inverting the reconstructed forward projected data.

14. The system (100) of any of claims 12 or 13, wherein the reconstructed volume of interest and the reconstruction of forward projected data are performed using FDK.
